(19) Europäisches Patentamt
European Patent Office
Office européen des brevets

(11) EP 1 565 565 B1

(12) EUROPEAN PATENT SPECIFICATION

(45) Date of publication and mention of the grant of the patent:
**05.08.2009 Bulletin 2009/32**

(21) Application number: **03775567.5**

(22) Date of filing: **18.11.2003**

(51) Int Cl.:
***C12Q 1/00*** *(2006.01)*

(86) International application number:
**PCT/GB2003/004984**

(87) International publication number:
**WO 2004/048603 (10.06.2004 Gazette 2004/24)**

(54) **SOL-GEL BIOSENSORS**

SOL-GEL-BIOSENSOREN

BIOCAPTEURS SOL-GEL

(84) Designated Contracting States:
**AT BE BG CH CY CZ DE DK EE ES FI FR GB GR HU IE IT LI LU MC NL PT RO SE SI SK TR**

(30) Priority: **25.11.2002 GB 0227424**

(43) Date of publication of application:
**24.08.2005 Bulletin 2005/34**

(73) Proprietor: **UNIVERSITY OF WARWICK**
**Coventry CV4 7AL (GB)**

(72) Inventors:
- **DALE, Nicholas, Egerton,**
**University of Warwick**
**Coventry CV4 7AL (GB)**
- **LLAUDET, Enrique,**
**University of Warwick**
**Coventry CV4 7AL (GB)**
- **DRONIOU, Magali,**
**University of Warwick**
**Coventry CV4 7AL (GB)**

(74) Representative: **Elsy, David et al**
**Withers & Rogers LLP**
**Goldings House**
**2 Hays Lane**
**London SE1 2HW (GB)**

(56) References cited:
**WO-A-99/10743** **US-B1- 6 303 290**

- **AVNIR D ET AL: "ENZYMES AND OTHER PROTEINS ENTRAPPED IN SOL-GEL MATERIALS" CHEMISTRY OF MATERIALS, AMERICAN CHEMICAL SOCIETY, WASHINGTON, US, vol. 6, 1994, pages 1605-1614, XP000938822 ISSN: 0897-4756**
- **SHACHAM R ET AL: "ELECTRODEPOSITION OF METHYLATED SOL-GEL FILMS ON CONDUCTING SURFACES" ADVANCED MATERIALS, VCH VERLAGSGESELLSCHAFT, WEINHEIM, DE, vol. 11, no. 5, 22 March 1999 (1999-03-22), pages 384-388, XP000827258 ISSN: 0935-9648 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 565 565 B1

Printed by Jouve, 75001 PARIS (FR)

## Description

The invention relates to methods of making sol-gel films on substrates, and the use of such films in, for example, biosensors and microarrays.

Sol-gels have been known about for over a hundred years, although their mechanism of formation was not explored until relatively recently. They have been used for a wide variety of applications, including optical coatings, dielectric semi-conducting uses and protective coatings. It is now known that it is possible to form films by direct usage of colloidal metal hydroxides, metal alkoxides or their pre-hydrolysed monomers. Dip coating of substrates is still normally used to coat substrates.

Dip coating involves the dipping of a substrate into a mixture containing the sol. The sol-gel formed will vary in thickness depending on a wide variety of factors including the speed of the withdrawal of the substrate from the mixture and the viscosity of the mixture.

Spin coating involves spinning the substrate on a turntable. A drop of sol mixture is dropped onto the spinning substrate and is dispersed across the surface of the substrate by centrifugal force, the disadvantage of this technique is that it is hard to control the final thickness of the sol-gel coating and is hard to produce a uniform thickness across the surface of the substrate.

Jones W.M. and Fischbach (D.B.), J. Non-Crystalline Solids (1988), Vol. 101, pages 123-126 disclose the production of silica hydrogels from acidified tetraethoxysilane (TEOS). This material forms sol-gels at room temperature, but takes several days to several weeks. The authors show that it is possible to increase the rate of sol-gel formation by adding dilute ammonium hydroxide. However, this is still stated to take several minutes at room temperature. Furthermore, no control of the position of the gel was disclosed.

US 5,698,083 discloses a sensor for detecting urea. This uses a layer of a sol-gel containing urease trapped within the sol-gel matrix. Entrapment of the urease is stated to be by "standard methods", that is dipping, spraying, painting etc.

Lillis B et al (Sensors and Activators B68 (2000), pages 109-114) disclose lactate oxidase immobilisation in four different sol-gel matrices. Glucose oxidase was also immobilised in such matrices. The sol-gels improved the stability of the enzymes. Sol-gels were prepared by aliquoting enzyme-containing sol-gel solutions into a 96-well plate and drying onto a membrane. The disadvantage of such a method is that there is little control over the eventual thickness and uniformity of the sol-gel layer.

Sol-gels have also been used to immobilise bacteria for virus particle detection (Black E. et al, J. Non. Cryst. Solids (2001), vol 285, pages 1-3).

The problem with conventional techniques of producing sol-gel layers is that they take a relatively long time to produce. They are also difficult to control to produce a uniform and reproducible layer. Conventional techniques, such as dipping and spin-coating also prevent the accurate deposition of small areas of sol-gels. This prevents the conventional technology being used in areas where reproducibility or spatial control is critical, such as the formation of bioassays, such as microarrays, and biosensors, such as microbiosensors.

Biosensors contain an electrode with a biorecognition molecule such as such as enzymes, antibodies, organelles and whole cells to allow the specific identification of analytes such as metabolytes, ions, gases and organic vapours. Such biorecognition elements are typically attached to a transducer, such as an electrochemical, piezoelectric, optoelectronic, fibreoptic, thermister, diode or surface acoustic device, to enable the binding of the analyte to the biorecognition element to be detected. Amperometric biosensors, for example, are reviewed in the article by Palmisano F., et al. (Fresenius J. Anal. Chem. (2000), Vol. 366, pages 586-601). A wide range of mediator compounds and mediator polymers have been used to enable the transfer of electrons from a suitable biorecognition element, such as an enzyme, to an electrode.

EP 0537761 discloses a biosensor comprising an electrode on a substrate of polyethylene tetraphthalate. An enzyme, such as xanthine oxydase is attached to an electrode via an electron acceptor, such as potassium ferricyanide.

Burmister J.J. and Gerhardt G.A. (Anal. Chem. (2001), Vol. 73, pages 1037-1042) discloses a ceramic-based multisite electrode comprising a plurality of separate electrochemical sensors arranged along the length of a needle micro electrode. A ceramic material is used as the base for such an electrode and the micro electrode is cut out by means of a laser.

Screenivas G., et al. (Anal. Chem. (1996), Vol. 68, pages 1858-1864) discloses the fabrication of sputtered-carbon micro electrode comprising a series of electrochemical sensors arranged along the length of a needle microprobe.

Microarrays are an orderly arrangement of samples, on, for example, a microplate or a membrane. The samples may be any of a variety of biological materials, including DNA, RNA, proteins, peptides, antibodies, viruses or even whole cells. The samples on microarrays are typically 200 microns in diameter. Microarrays are typically used to screen, for example, several genes at once.

Sol-gels have potential for improving the stability of biological material and for filtering materials prior to contacting the biological material. However, conventional methods of sol-gel deposition do not allow the spatial resolution

for the production of such microarrays.

Sol-gel films have been prepared by electrodeposition and electrochemical techniques. The electrodeposition of sol-gels is described in the article by Shacum R., et al. (Adv. Mater (1999), Vol. 11 (5), pages 384-388.

The Shacum paper describes a method of the electrodeposition methyltrimethoxysilane (MeTMOS) on indium-tin-oxide. The authors of the paper use an acidified suspension of MeTMOS in a solution of ethanol and potassium nitrate, buffered to pH 3.5 with phthalate buffer. The indium-tin-oxide electrode was placed in the solution for up to 30 minutes whilst an electrical potential was applied to the electrode. The electrical potential was observed to cause a layer of sol-gel to be deposited on the surface of the electrode. The reduction of nitrate on the surface of the electrode was thought to cause a localised increase in pH at the electrode surface according to equation 2 (below). Nitrate is reduced by the electrical current at the electrode to produce hydroxide ions:

$$1)\quad H^+ + e^- \rightarrow \tfrac{1}{2} H_2$$

$$2)\quad NO_3^- + 6H_2O + 8e^- \rightarrow NH_3 + 9OH^-$$

This was thought to cause the catalysis of the formation of the sol-gel at the electrode surface. The reaction is complex but was thought to be able to be summarised as:

$$MeSi(OMe)_3 + H_2O \rightarrow MeSi(OH)_3 + MeOH \text{ (unbalanced)}$$

$$MeSi(OH)_3 \rightarrow Me_nSi_nO_pH_q + H_2O \text{ (unbalanced)}$$

$$\text{(sol-gel)}$$

The inventors have realised that the selective electro-deposition of sol-gels could be a way of selectively depositing a layer of biorecognition molecules onto a surface to form, for example, a biosensor. However, the low pH and the relatively long application of the electrical potential tends to denature biorecognition elements within the sol-gel resulting in very poor results. Neutralising the acidified sol suspension, prior to applying an electrical potential to the electrode, has unexpectedly been found to allow the successful application of biorecognition elements, without their substantial denaturation. This also unexpectedly reduces the amount of time that it takes to deposit the sol-gel on the electrode, therefore decreasing production time. The formation of sol-gels by electrodeposition also allows the thickness of the layer to be controlled. The position of the sol-gel can be selected by applying an electrical potential to a specific part of a substrate allowing selective deposition on e.g. a biosensor or microarray. These advantages can be applied to various uses of sol-gels including the production of biosensors and microarrays. Where biosensors are made, increases in sensitivity over known methods have been observed. Furthermore, this technology allows, for example, microarrays and biosensors to be made relatively inexpensively.

A further advantage of this approach is stock solutions of the acidified sol may be made up and stored until needed. The remaining ingredients, such as enzymes, may be added as and when a specific electrode needs to be produced. This allows greater flexibility in the production of, for example, sensors or microarrays.

The invention provides a method of producing a layer of a sol-gel on a substrate comprising the steps of

(a) providing an acidified sol suspension;

(b) at least partially neutralising the acidified sol suspension to form a neutralised sol suspension;

(c) contacting an electrically conductive surface with the neutralised sol suspension; and

(d) applying negative electrical potential to the electrically conductive surface to cause a layer of sol-gel to form on the surface of the electrically conductive surface.

By sol-gel we mean a colloidal suspension of sol particles that is gelled to form a solid. A sol is made of colloidal particles which are, prior to gelling, dispersed in a fluid such as liquid. Typically colloidal particles have a size of 1 nm

to 100 nm diameter.

The acidified sol suspension contains the sol-gel monomer that condenses on applying the electrical potential to create the sol-gel.

Preferably the neutralised sol suspension has one or more biological material elements added prior to applying the electrical potential. Such biological material includes proteins such as enzymes and antibodies, and fragments of antibodies such as Fab and $F(ab^1)_2$, nucleic acids (such as DNA, RNA or oligonucleotides), organelles, peptides, polysaccharides, oligosaccharides, biomimetic polymers, viruses, microorganisms, and whole eukaryotic or prokaryotic cells. Most preferably, the biological material is an enzyme.

Potentially any biological material may be used. A mixture of such materials may be used. Microarrays containing different biological materials are well known in the art, and the technology of the invention may be applied to the production of such microarrays or indeed larger biological assays, such as immunoassays. Immunoassays are used for a wide variety of assays, including the assay of hormones associated with pregnancy.

Preferably, no alcohol, such as ethanol or methanol, is added to the sol, thereby increasing the stability of the biorecognition molecule. If alcohol is added, it is preferably only added in an amount sufficient to solubilise the silane.

Microarrays or other biological assays may contain, for example, nucleic acid probes, such as DNA probes immobilised in the sol-gel. Proteins, such as enzymes and receptors, peptides, aptamers or substrates for example enzymes or antigens, for assaying antibodies may also be incorporated. Ligands for assaying for the interaction with peptides may also be provided. Alternatively, peptides may be provided, for example to assay for, or study peptide-ligand interactions. Small molecules, such as co-factors, colorimetric molecules, fluorescent molecules and luminescent molecules (such as entrapped dyes) may be incorporated into the sol-gel. The use of microarrays is reviewed in Templin M. Weld (Trends in Biotechnology (2002), Vol. 20, pages 160-165) and Angenendt P et al (Anal. Biochem. (2002) vol 309, pages 253-260).

Enzymes may be used to form for example biosensors.

Preferably, the enzyme is selected from one or more of xanthine oxidase, glucose oxidase, lactate oxidase, cholesterol oxidase, galactose oxidase, glutamate oxidase, horse radish peroxidase, polyphenol oxidase, D-fructose dehydrogenase, L-glutamate dehydrogenase, alcohol dehydrogenase (such as methanol dehydrogenase), urease, uricase, lactate dehydrogenase, glutamic pyruvic transaminase, creatinase, sarcosine oxidase, glutaminase, nucleoside phosphorylase, ascorbate oxidase, cytochrome C oxidase, adenosine deaminase, D- or L-amino acid oxidase, tyrosinase, catalase, phosphoenolpyruvate kinase, glycerol kinase, glycerol-3-phosphate oxidase, phosphocreatine kinase, acetylcholine esterase, choline oxidase and/or cholinedehydrogenase. Other enzymes known in the art may also be used. The enzymes may be used separately or two or more together in the form of a cascade to measure one or more different substrates. Typically, such electrodes use an oxidoreductase which is capable of transferring an electron, preferably via a mediator, onto the electrode. Alternatively, the enzyme may be capable of receiving an electron from the electrode, optionally via a suitable mediator.

Most preferably the enzymes used are:

Glucose oxidase - to measure glucose in, for example blood.

A cascade containing creatinine amidohydrolase (EC 3.5.2.10), and creatine amidohydrolase (EC 3.5.3.3) and sarcosine: oxygen oxidoreductase (demethylating)(EC 1.5.3.1), may be used to measure creatinine, e.g. in blood. These enzymes convert creatinine to creatine, creatine to sarcosine, and sarcosine to glycine, formaldehyde and hydrogen peroxide respectively. Hydrogen peroxide is measured at an electrode surface.

Cholesterol may be measured using steryl-ester acyl hydrolase (EC 3.1.1.13) and cholesterol: oxygen oxidoreductase (EC 1.1.3.6) to convert cholesterol ester to amongst other products, detectable hydrogen peroxide.

Triglycerides may also be measured by converting them to detectable hydrogen peroxide using a cascade of Lipoprotein Lipase (EC 3.1.1.34), ATP : glycerol 3-phosphotranferase (EC 2.7.1.30) and glycerol 3-phosphate oxidase.

Adenosine may use adenosine deaminase, nucleoside phosphorylase and xanthine oxidase to detect it.

ATP may be detected with glycerol kinase and glycerol-1,3-phosphate oxidase. Enzymatic amplification can be achieved by including creatine kinase as well.

Xanthine oxidase may be used, for example, to detect hypoxanthine. If xanthine oxidase is used together with nucleoside phosphorylase, then the electrode may be used to detect xanthine, inosine and hypoxanthine. The addition of adenosine deaminase to the enzymes converts purines, such as adenosine, into inosine, therefore allowing the detection of purines such as adenosine by the sensor.

Most preferably, one or more enzymes is added. The ability to induce several enzymes at the same time improves the speed of production of the biosensor and the response obtainable by such biosensors.

The enzymes may produce a diffusable molecule, such as $H_2O_2$ which is detected at the electrode. Alternatively, a mediator may be used to transfer electrons to or from the electrode of the biosensor.

Other enzymes include Rnase, DNase, nuclease, ribonuclease and catalase. Active proteins, such as haemoglobin, myoglobin, collagen or tubulin may be added. Antibodies or fragments of antibodies, such as IgG, IgM or Fab or $F(ab^1)_2$ fragments may also be incorporated.

Preferably, the acidified suspension has a pH of less than pH 4, especially less than or equal to pH 3.5. That is, the starting pH on mixing the silane and acid, preferably prior to sonication, is preferably less than pH 4.

The pH of the neutralised sol suspension is preferably pH 5 to pH 7.5, more preferably pH 5.5 to pH 7.0, or pH 6.0 to pH 6.5, especially pH 6.3. This is preferably neutralised by the addition of a suitable buffer such as HEPES, PIPES or MOPS buffer. This allows the pH to be accurately controlled.

The sol preferably comprises a sol of alkoxysilane, alumina or colloidal metal hydroxide.

US 6303290 discloses a ceramic oxide colloidal sol which may be mixed with an acidified salt solution. Adding hydroxide increases the pH causes the formation of a sol gel. Using the method of the invention, instead of hydroxide, is expected to allow the electrodeposition of the sol-gel onto a substrate.

Accordingly, preferably the sol is a ceramic oxide sol, such as titanium oxide.

Zirconia ceramics may also be used as the sols. Gheorghies C et al (Analele Stiintifice Ale Universitatii, Tomul XLV - XLVI, s. Fizica Starii Condensate (1999-2000) pages 268-275) discloses cathodic depositions of Zirconia sol-gels. The electrical current was used to produce hydroxide ions which caused the deposition of the film. Preferably the acidified sol is Zr $O(NO_3)$.

Preferably the sol is a silane. Preferably it has a general formula:

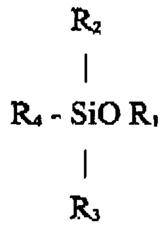

or $(R_9O)_3$-Si-$CH_2$-$CH_2$-Si-$(OR_9)_3$
where:
$R_1$ = straight chain, branched chain, cyclic, non-cyclic, saturated or non-saturated, substituted or non-substituted alkyl; substituted or non-substituted aryl; -$NR_5$; and -$COR_6$; the alkyl preferably contains 1, 2, 3, 4, 5 or 6 carbon atoms
$R_2$, $R_3$ and $R_4$ arc independently selected from; straight chain, branched chain, cyclic, non-cyclic, saturated or non-saturated alkyl; -$COR_6$; -O-alkyl; and -O-$COR_6$; -$R_7R_8$; $R_7N(R_6)_2$ and $R_7NHR_6R_8$; preferably containing 1, 2, 3, 4, 5, or 6 carbon atoms.
$R_5$ = branched or non-branched cyclic or non-cyclic, saturated or non-saturated alkyl; or

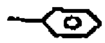 ,

preferably containing 1, 2, 3, 4, 5, or 6 carbon atoms;
$R_6$ = $C_1$ to $C_3$ alkyl;
$R_7$ = $C_1$ to $C_6$ alkyl, especially $C_1$, $C_2$ or $C_3$ alkyl;
$R_8$ = Epoxy, -$NH_2$ or -SH; especially

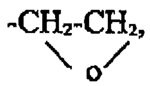 or 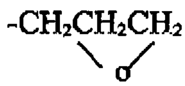

$R_9$ = Straight or branched $C_1$ to $C_6$ alkyl.

Preferably $R_1$ is methyl, ethyl, propyl, -$NCHCH_2CH_3$, -$NC(CH_3)CH_2CH_3$, -$NC(CH_3)CH_2CH(CH_3)_2$, -$COCH_3$ or - N =

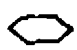

Preferably $R_2$, $R_3$ and $R_4$ are independently, methyl, ethyl, propel, -O-ethyl, -O-ethyl, -O-propyl, -$CHCH_2$,

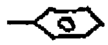

or $-OCOCH_3$.

Preferably the silanes are selected from:

Tetra methoxysilane (TMOS), tetra ethyoxysilane (TEOS), tetrapropoxisilane, methyltrimethoxysilane, methyltriethoxysilane, methyltris(methyl-ethylketoxime) silane (MOS), methyl tris(acetoxime) silane, dimethyldi(methylethylketoxime) silane, trimethyl(methylethylketoxime) silane, vinyl tris (methylethylketoxime) silane (VOS), methyl tris (methylisobutylketoxime) silane, methylvinyl di (methylethylketoxime) silane, methyl-vinyldi (cyclo hexanoneoxime) silane, vinyl tris (methyl isobutylketoxime) silane, phenyltris(methylethyl ketoxime) silane (POS), methyl triacetoxysilane and tetraacetoxysilane.

Preferably:

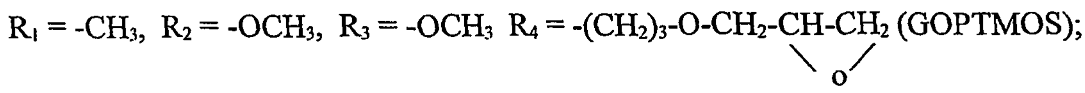

$R_1 = -CH_3$, $R_2 = -OCH_3$, $R_3 = -OCH_3$, $R_4 = -(CH_2)_3NH_2$ (APTMOS);

$R_1 = -CH_2CH_3$, $R_2 = -OCH_2CH_3$, $R_3 = -O-CH_2CH_3$, $R_4 = -(CH_2)_3NH_2$ (APTEOS);

$R_1 = -CH_3$, $R_2 = -OCH_3$ $R_3 = -OCH_3$, $R_4 = -(CH_2)_3SH$ (MPTMOS);

$R_9$ = ethyl (Bis TEOS); or $R_9$ = methyl (Bis TMOS)

Most preferably the silane is tetramethoxysilane (TMOS).

Such silanes are known in the art and available commercially.

Two or more silanes may be used.

Preferably a silane coupling agent is added. Such coupling agents contain a reactive group such as an amine, sulphydryl, oxy, acrylate, vinyl or chloro group. This may be to the acidified sol suspension or to the neutralised sol suspension. These have been found to improve the stability of the film. Preferred coupling agents include:

Aminopropyltriethoxysilane
Aminopropyltrimethoxysilane
Aminopropylmethyldiethoxysilane
Aminopropylemthyldimethoxysilane
Aminoethylaminopropyltrimethoxysilane
Aminoethylaminopropyltriethoxysilane
Aminoethylaminopropylmethyldimethoxysilane
Diethylenetriaminopropyltrimethoxysilane
Diethylenetriaminopropyltriethoxysilane
Diethylenetriaminopropylmethyldimethoxysilane
Diethylenetriaminopropylmethyldiethoxysilane
Cyclohexylaminopropyltrimethoxysilane
Hexanediaminomethyldiethoxysilane
Anilinomethyltrimethoxysilane
Anilomethyltriethoxysilane
Diethylaminomethyltriethoxysilane
(Diethylaminomethyl)methyldiethoxysilane
Methylaminopropyltrimethoxysilane
Bis(triethoxysilylpropyl)tetrasulfide
Bis(triethoxysilylpropyl)disulfide
Mercaptopropyltrimethoxysilane
Mercaptopropyltriethoxysilane
Mercaptopropylmethyldimethoxysilane
3-thiocyantopropyltriethoxysilane
Glycidoxypropyltrimethoxysilane

Glycidoxypropyltriethoxysilane
Glycidoxypropylmethyldiethoxysilane
Glycidoxypropylmethyldimethoxysilane
Methacryloxypropyltrimethoxysilane
Methacryloxypropyltriethoxysilane
Methacryloxypropylmethyldimethoxysilane
Chloropropyltrimethoxysilane
Chloropropyltriethoxysilane
Chloromethyltriethoxysilane
Chloromethyltrimethoxysilane
Dichloromethyltriethoxysilane
Vinyltrimethoxysilane
Vinyltriethoxysilane
Vinyltris(2-methoxyethoxy)silane
Trimethoxysilyl aminopropyl aminoethyl triacetic acid (MeO) 3SiCH2CH2CH2N (CH2COOH) CH2CH2N (CH2COOH)2

An electrical potential of -900 to -1500 mV, especially -900 to -1200 mV, is preferably used. The electrical potential may be applied for 10 to 120 seconds, especially 20 to 60 seconds, most preferably 20 to 40 seconds, depending on the thickness of sol-gel required.

Preferably the coupling agent is 3-aminopropyl-trimethoxysilane (APTEOS)

Coupling agents which have amino groups may be functionalised, e.g. by adding ferrocene or lacto bionamide or gluconamide. This allows the electron transport ability of the sol-gel to the electrically conductive layer to be improved or the stability of the gel to be modified.

Glycidylpropyltrimethoxysilane may also preferably be added to the neutralised sol-suspension.

The silane coupling agents preferably comprise one or more, especially two or more reactive groups, such as amine groups that cross-link the silane moieties.

Initial results show that the presence of mercaptan-containing silanes, such as MPTMOS, improve the sensitivity of biosensors produced by this technique, for example by lowering the sensitivity of the biosensor to contaminants in an assay mixture. They also improve the stability of biosensors in storage.

Initial results also show that the addition of bisfunctional silanes, such as Bis TEOS or Bis TMOS, improve the stability and mechanical strength of biosensors.

The inventors have found that omitting alcohols, such as ethanol and/or electroreducers, such as nitrate, from the acidified sol suspension improves the stability of the suspension, allowing to be stored for longer. Such alcohols or electroreducers are usually added to the neutralised sol suspension, for example prior to or after the addition of alkali such as buffer, allowing it to be stored longer.

The electroreducer is one which reduces to form hydroxide ions or other such catalytic ions, upon application of the electrical potential. Preferably the electroreducer is nitrate.

The inventors have found that the sol-gel may be stabilised by the addition of one or more stabilisers such as a polyhydroxyalcohol. Examples include glycerol, polyethylene glycol (PEG) or polyvinyl alcohol; polysaccharides, such as dextran or chitosan; polyalkylene imine; or sugars, such as mannitol, gluconate, gluconolactone, trehalose, lactitol or sucrose.

Two or more enzymes may be used in a cascade; such enzymes may be mixed together and applied at the same time. Alternatively they may be applied as separate layers. A first layer is applied by switching on the electrical potential to form a first layer of sol-gel. The electrically conductive substrate is placed in a second neutralised sol suspension containing a second enzyme, and a second sol-gel layer is applied by switching on an electrical potential again. The sol used in the second suspension may be the same as or different to the first suspension.

The electrically conductive surface may be carbon or a metal or metal alloy such as carbon paste, gold, platinum, or platinum-iridium alloy. This may form a part of a biological assay device, such as an electrode for a biosensor or a substrate for a microarray.

The substrate may be an array of microneedles (Discover (1998) Vol 19). These are small needles that can be used to penetrate the skin with little or no pain to a patient. The method of the invention may be used to form a biosensor using the needles as electrodes or to put other biological compounds on the needles to allow them to assay for analytes in a patient's body. This would be used, for example as a painless glucose sensor for diabetics.

Two different electrodes may be provided, each electrode having a layer of sol-gel having a different biorecognition element within it, formed by applying an electrical potential to a first electrode when in contact with a first neutralised sol suspension containing a first biological material; and
selectively applying an electrical potential to a second electrode, when the second electrode is in contact with a second

neutralised sol suspension containing a second biological material.

Accordingly, the invention provides a method of producing a biological assay device, such as a biosensor or microarray comprising the step of producing a layer of sol-gel on an electrically conductive substrate by the method of the invention.

The layer of sol-gel containing the biological material may be covered by an additional layer of sol-gel that does not contain biological material. This additional layer may be used to filter out one or more impurities before they reach the sol-gel layer containing the sol-gel containing the biological material or simply to provide a protective layer.

The invention therefore provides a method according to the invention, additionally comprising the steps of:

(e) providing an acidified sol suspension without any biological material; and

(f) applying a negative electrical potential to the electrically conductive surface to cause a further layer of sol-gel to form.

This further layer will be separated from the electrically conductive layer from the first layer of sol-gel formed. The second layer need not be neutralised as no biological material is used. However, neutralising the acidified sol suspension may be used to increase the rate of gel formation and to preserve biological material in the existing layer.

Microarrays or biosensors may be formed by forming, for example by printing or by producing using microprocessor technology such as CMOS, an electrically conductive layer on a substrate. If several different areas of conductive layer are formed then each layer may be separately subjected to a charge when the substrate is placed on different sol-gel suspensions. This allows the formation of different areas sol-gels containing different biological material.

The method of the invention may be used to form an immunoassay device. In such a device the biological material is an antibody or a fragment of an antibody, such as a Fab or $F(ab^1)_2$ fragment. This may be used, for example with a labelled antigen (for example labelled with a radiolabel or fluorescent label) to form competition assay to measure the presence or absence of our antigen of interest.

Accordingly, preferably the method of the invention is used to produce an immunoassay device.

Preferably the biological material is an antibody or a fragment of an antibody capable of binding a predetermined antigen, such as an Fab or $F(ab^1)_2$ fragment.

The layer of sol-gel on its substrate may be washed with, for example, water or buffer.

Microelectrode biological assay devices, as defined in claim 26, comprising one or more biological meterials, are provided. They may be used in combination with a potentiometer to detect electrical output from the sensor.

Preferably the sensor is a microelectrode, for example of the type known in the art. The microelectrode substrate may be made from silicon. Silicon-based microelectrodes are the subject of a copending application.

Such microelectrodes need to be able to have a layer containing a biorecognition element selectively applied to the small area of the electrode. The method of the invention allows this to be achieved relatively easily and controllably. Microelectrodes are typically less than 10 mm long, most preferably 0.5 - 2 mm long and/or 10 - 50 µm in diameter, which make methods of producing sol-gel layers difficult to control. The methods of the invention alleviate this problem.

The use of biosensors, microarrays or immunoassays made by the invention to detect analytes are also provided.

Preferably, the analyte is selected from the following analytes, which are given below, together with the preferred combination of enzymes used for their detection:

glucose (glucose oxidase), lactate (lactate oxidase), cholesterol (cholesterol oxidase), galactose (galactose oxidase), glutamate (glutamate oxidase), hypoxanthine (xanthine oxidase), hydrogen peroxide (horse radish peroxidase), fructose (D-fructose dehydrogenase), glutamate (L-glutamate dehydrogenase), ethanol (alcohol dehydrogenase), methanol (methanol dehydrogenase), urea (urease), uric acid (uricase and horse radish peroxidase), lactate (L-lactate dehydrogenase and glutamic pyruvic transaminase), creatine (creatininase and creatinase and sarcosine oxidase), glutamine and glutamate (glutaminase or glutamate oxidase).

Most preferably the enzymes used are:

Glucose oxidase - to measure glucose in, for example blood.

A cascade containing creatinine amidohydrolase (EC 3.5.2.10), and creatine amidohydrolase (EC 3.5.3.3) and sarcosine: oxygen oxidoreductase (demethylating)(EC 1.5.3.1), may be used to measure creatinine, e.g. in blood. These enzymes convert creatinine to creatine, creatine to sarcosine, and sarcosine to glycine, formaldehyde and hydrogen peroxide respectively. Hydrogen peroxide is measured at an electrode surface.

Cholesterol may be measured using steryl-ester acyl hydrolase (EC 3.1.1.13) and cholesterol: oxygen oxidoreductase (EC 1.1.3.6) to convert cholesterol ester to amongst other products, detectable hydrogen peroxide.

Triglycerides may also be measured by converting them to detectable hydrogen peroxide using a cascade of Lipoprotein Lipase (EC 3.1.1.34), ATP : glycerol 3-phosphotranferase (EC 2.7.1.30) and glycerol 3-phosphate oxidase.

Adenosine may use adenosine deaminase, nucleoside phosphorylase and xanthine oxidase to detect it.

ATP may be detected with glycerol kinase and glycerol-1,3-phosphate oxidase.

Xanthine oxidase may be used, for example, to detect hypoxanthine. If xanthine oxidase is used together with nucleoside phosphorylase, then the electrode may be used to detect xanthine, inosine and hypoxanthine. The addition of adenosine deaminase to the enzymes converts purines, such as adenosine, into inosine, therefore allowing the detection of purines such as adenosine by the sensor.

The sol-gel is preferably obtainable from one or more silanes and contains one or more biological materials as defined above and may contain one or more additives as defined above.

Preferably, sol-gel is obtainable from a mixture comprising a mercapto-containing silane and/or a bisfunctional silane.

The invention will now be described by way of example only, with reference to the following figures:

**Figure 1** shows the response of a sensor made by an alternative technique.

**Figure 2** shows the response of a sensor made, using the method of the invention.

**Figure 3** shows the response of a sensor made using an alternative method of the invention.

1. **COMPARATIVE METHODS**

The three enzymes, adenosine deaminase (AD), nucleoside phosphorylase (PNP) and xanthine oxidase (XO), were purchased from Sigma. Physiological saline containing 115 mM NaCl, 2.4 mM $NaHCO_3$, 10 mM HEPES, 3 mM KCl, 1mM $MgCl_2$, 2mM $CaCl_2$, 1mM $NaH_2PO_4$, 1mM $Na_2HPO_4$, at pH 7.4 was used in all experiments to test and use the sensor.

**Synthesis of amphiphillic pyrrole (monomer 1): (12-Pyrrol-1-yldodecyl)triethylammonium Tetrafluoroborate**

Briefly, potassium metal (1 molar eq.) was added in small pieces to a solution of pyrrole (0.97 molar eq.) in dry THF, the mixture was stirred under nitrogen for 12 hours. After filtration the yellow solid was washed with cold THF and vacuum dried. Pyrrolyl potassium (I) (1 molar eq.) and 12-bromododecanol (0.5 molar eq.) were refluxed for 30 min in a mixture of dry THF and dry DMSO (4:1). The resulting solution was diluted with water and extracted with DCM. The organic phase was dried over Na2SO4 and rotary evaporated. The desired product was purified by chromatography on a silica column eluted with a 1:1 heptane-diethylether mixture. A solution of 12-pyrrol-1-yldodecan-1-ol (II) (1 molar eq.) and tosyl chloride (1 molar eq.) in anhydrous pyridine was stirred at 5°C for 12 hours. The mixture was poured into water and extracted with diethylether. The organic phase was washed four times with 5% HCl aqueous solution and once with water; after drying over Na2SO4 the solvent was removed under reduced pressure. The desired product was purified by chromatography on a silica column eluted with a 1:1 heptane-diethylether mixture. 12-Pyrrol-1-yldodecyl *p*-Toluenesulfonate (III) (1 molar eq.) was refluxed for 24 hours in dry ethanol in the presence of triethylamine (3 molar eq.). After vacuum evaporation of the solvent and excess amine the product was purified by chromatography on a silica column eluted with a 9:1 CH3CN-H2O mixture. (12-Pyrrol-1-yldodecyl)triethylammonium Tosylate (IV) was dissolved in 1:1 water-methanol mixture (~10ml / 100mg), the solution was then stirred with anion-exchange resin (Amberlite IRA 900-Cl) in BF4- for 1 hour. After filtration this process was repeated four times. Evaporation of the solvent by freeze-drying gave the desired product as white powder. Final purification by chromatography on a silica column eluted with a 9:1 CH3CN-H2O mixture lead to the pure desired product (V).

$^1$H NMR ($CDCl_3$) d (ppm) 1.3 (m, 25 H, H-3, H-7), 1.60 (m, 2 H, H-4), 1.70 (t, *J* = 7 Hz, 2 H, H-2), 3.10 (q, *J*= 3.5 Hz, 2 H, H-5), 3.3 (m, 6 H, H-6), 3.85 (t, *J* = 7.6 Hz, 2 H, H-1), 6.10 (t, *J*= 1.5 Hz, 2 H, H-a), 6.6 (t, *J*= 1.5 Hz, 2 H, H-b); MS (TOF) m/z, $M^+$, 335.

**Synthesis of the Lactobionamide pyrrole (monomer 2): 8-Pyrrol-1-lactobionamidooctane**

In brief, 8-Pyrrol-1-yloctacyl *p*-Toluenesulfonate (I) (1 molar eq.) was refluxed in dry DMF with sodium azide (5 molar eq.) until the reaction was complete. After removal of the solvent under reduced pressure, the residue was dissolved in Et2O and the insoluble salts were filtered off. After vacuum evaporation of the solvent the azide intermediate (II) was obtained as an orange oil. This product was then treated with an excess of Dithiothreitol (5 molar eq.) in DMF and triethylamine (5 molar eq.) for 30 min at R.T. The resulting mixture was then poured into water and extracted with Et2O. After drying the solvent over Na2SO4 and rotatory evaporation the desired product was obtained as a yellow oil.

8-Pyrrol-1 aminooctane (III) (1 molar eq.) was added to a solution of lactobionic acid (1 molar eq.) in methanol, the mixture was refluxed for 24 hours. The solvent was evaporated under vacuum and the product was obtained as a pale yellow powder (IV).

$^1$H NMR ($CDCl_3$) d (ppm) 1.25 (m, 12 H, H-2), 1.45 (m, 2 H, H-3), 1.7 (m, 2 H, H-1), 3-5.5 (m, 23 H, H-5), 6.1 (m, 2 H, H-a), 6.6 (m, 2 H, H-b), 7.8 (t, 1 H, H-4); MS (TOF) m/z, M+Na, 557.

**Solubilization of monomers**

Monomer 1 solution was made by mixing 5 mg of monomer with 1 ml of water and vigorously stirring with a vortex, the mixture was then sonicated for 5 minutes, after adding 10 % (v/v) $CH_3CN$ and mixing again a white suspension was obtained. Electropolymerization of monomer 2 was performed in 0.1M $LiClO_4$ deaerated aqueous solution with 10% (v/v) $CH_3CN$.

**Apparatus**

A potentiostat (Model AEW-2) from Sycopel was used to electrochemically deposit the different polymers and test the sensor. The sensor was used *in vivo* with a World Precision Instruments *Micro* C potentiostat interfaced to a PC by an A to D converter board (Data Translation). In all cases an Ag/AgCl was used as reference electrode; no counter electrode was needed due to the small size of the working electrode. The electrochemical cell for deposition consisted of a capillary of 1.5 mm diameter and 2 cm length.

**Sensor fabrication**

Assembly and cleaning

The microelectrode was assembled by soldering 2 cm of sensing wire to a copper wire with a terminating pin. Initially 250 mm pure Pt wire was used, which was subsequently etched to the desired final diameter. However pure Pt wire is very soft, limiting the smallest usable diameter to around 50 mm. To make sensors of even smaller diameter Pt/Ir wire (90/10 from Goodfellow Metals) in diameters ranging from 25 to 100 mm. was also used. This Pt/Ir wire is much stiffer, yet can still be used to make highly sensitive electrochemical sensors.

All but the final 2 mm of the Pt wire was protected by a pulled glass capillary that was fused by heat to the wire. The central part of the assembly was insulated with heat shrink tubing. The exposed tip of Pt was then etched under visual inspection by 1.2 V AC electrolysis in 2M NaCl using a spiral Pt coil as counter electrode (Slevin, *et al.*, 1999). The final diameter ranged from 25 mm to 100 mm depending on the extent of etching. Note that the 25 mm Pt/Ir wire was sufficiently small to be used without further etching. In addition we found that excessive etching of the Pt/Ir wire produced a surface unfavorable for polymer deposition.

The exposed electrode was then coated with Sylgard (resin 184, Dow-Corning) to leave a final length of exposed Pt. This length was varied to suit the experimental requirements and we have constructed sensors that range in length from 300 mm to 2mm. Careful surface preparation of the Pt electrode was crucial to the ability to deposit the pyrrole polymer and to the overall sensitivity of the sensor. Without careful cleaning of the Pt surface, the polymer layers would not grow sufficiently well to entrap the enzymes efficiently. The Pt electrode was therefore cleaned by cycling in 0.1 M $H_2SO_4$ from -100mV to 1000mV (versus Ag/AgCl reference, scan rate 100 mV/s) for 15 times. Before polymer deposition the electrode was held at 1000 mV for 1 minute.

Deposition of polymers

All sensors involved a first layer of LBA derivative polymer formed by cycling the electrode 15 times from 0 to 800 mV, scan rate 100 mV/s in a de-aerated solution of monomer 2 (10 mM) in 0.1 M $LiClO_4$, 10% $CH_3CN$ (Fig 2). After this procedure the exposed Pt appeared black in colour. The unbound monomer was removed by washing the electrode in stirred $dH_2O$ for 5 min. To entrap the enzymes the electrode was then immersed in a solution of monomer 1 (5 mg/ml, 10% $CH_3CN$) containing the desired enzyme, and the potential held to 760 mV for 10 minutes. To save monomer and enzymes, the polymer deposition was carried in a mini-chamber of 10 mL volume consisting of a short capillary glass tube.

To make an adenosine sensor three layers were deposited from solutions of monomer 1, firstly 1 U of AD in 10 ml was used, followed by 1 U of PNP in 10 ml and then 5 U of XO also in 10 ml. This procedure could be optionally repeated to give a sensor of greater sensitivity. Before testing the sensor was stirred in phosphate buffer for 5 minutes to wash unbound monomer and enzyme. Null sensors were made by depositing the same first layer of LBA polymer followed by a second layer of amphiphilic polymer, deposited for 5 min. at 760 mV from a solution of monomer 1 with no enzymes

2. **METHOD OF THE INVENTION**

Solutions were made up in distilled water unless otherwise indicated.

(A) **Example 1**

(i) **Acidified TMOS suspension**

7.39 ml. TMOS
1.69 ml. distilled $H_2O$
0.11 ml. 0.04 M HCl
Sonicated on ice for 20 mins. stored on ice for up to several days.

(ii) **APTEOS suspension**

5.55 ml. APTEOS
5 ml. $H_2O$
pH adjusted to pH 3 with cone HCl and then sonicated on ice for 20 mins. prior to storing on ice.

(iii) **Neutralised sol suspension**

Mix:

1 part acidified TMOS suspension
1 part APTEOS
2 parts ethanol
2 parts 1M glycerol
4 parts Tris 50 mM pH 6.3
2 parts 0.4 M $KNO_3$
1 part PEG (400 av. mol. wt.)
1 part DEAE - dextran

This was stored on ice.

(iv) **Film formation**

Enzymes were usually added just before use. Film formation on the electrode, such as Pt or Pt/Ir alloy 20-40 seconds. Adenosine Deaminase, nucleoside phosphorylase and xanthine oxidase (AD, PNP and XO), in this particular example, were mixed together just prior to applying the electrical potential.

(B) **Example 2 - dual layer electrode**

A biosensor was constructed in a similar manner to Example 1, but using the following compounds:

| | Ratio (vol/vol) |
|---|---|
| Acidified TMOS | 0.6 |
| Acidified APTEOS | 0.1 |
| Acidified MTMOS | 0.3 |
| Lactitol (1M) | 1 |
| $Ca(NO_3)_2$(0.2M) | 1.5 |

(continued)

| | Ratio (vol/vol) |
|---|---|
| Tris (pH7, 50 mM) | 2 |
| PEG 400 (100%) | 1 |

MTMOS is methyltrimethoxysilane, acidified in the same manner as TMOS from Example 1.

2.5 U glycerol-3-phosphate oxidase was dissolved in 10 µl 10% Dextran (pH7) and 10 µl sol was added. This was deposited onto an electrode, such as a Pt or Pt/Ir for approximately 30 seconds to form a first layer of sol.

5 U glycerol kinase was dissolved in 10 µl PEG 400/1 M glycerol (50:50 by volume) and 10 µl sol was added. Deposition of the sol onto the first layer of sol was carried out for approximately 2 minutes.

**RESULTS**

Figure 1 shows typical results from another electrode system (~100 pA for 10 µM adenosine, ~200 pA for 10µM inosine and ~5nA for 10 µM xanthine).

The electrode of the invention produces typical read-outs as shown in Figure 2. The response was 10µM adenosine (~8 nA) and 10µM inosine (~9 nA). Increases in sensitivity of approximately 100-fold compared with the previous electrode was observed.

The second electrode results are shown in Figure 3 for 100 µM, 50 µM, 20 µM and 10 µM ATP.

**Claims**

**1.** Method of producing a layer of a sol-gel on a substrate comprising the steps of

(a) providing an acidified sol suspension;
(b) at least partially neutralising the acidified sol suspension to form a neutralised sol suspension;
(c) contacting an electrically conductive surface with the neutralised sol suspension; and
(d) applying an electrical potential to the electrically conductive surface to cause a layer of sol-gel to form on the surface of the electrically conductive surface.

**2.** A method according to claim 1, additionally comprising the step of adding one or more biological materials to the neutralised sol suspension, prior to applying the electrical potential to the electrically conductive surface (step C).

**3.** A method according to claim 2, wherein the biological material selected from an enzyme, antibody, fragment of an antibody, nucleic acid, polysaccharide, oligosaccharide, biomimetic polymers, virus, microorganism or a whole cell.

**4.** A method according to any preceding claims, wherein the acidified sol suspension has a pH of less than pH 4.

**5.** A method according to any preceding claim wherein the acidified sol suspension is neutralised to between pH 5 and pH 7.5.

**6.** A method according to any preceding claim wherein the acidified sol suspension is neutralised by the addition of a buffer.

**7.** A method according to any preceding claim wherein the sol comprises a sol of alkoxysilane, alumina, colloidal metal hydroxide, ceramic oxide or zirconia.

**8.** A method according to claim 7, wherein the sol has the general formula:

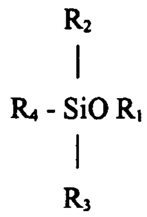

or $(R_9O)_3$-Si-$CH_2$-$CH_2$-Si-$(OR_9)_3$
where:

$R_1$ = straight chain, branched chain, cyclic, non-cyclic, saturated or non-saturated, substituted or non-substituted alkyl; substituted or non-substituted aryl; -$NR_5$; and -$COR_6$; preferably containing 1, 2, 3, 4, 5 or 6 carbons;
$R_2$, $R_3$ and $R_4$ are independently selected from; straight chain and branched chain, cyclic or non-cyclic, saturated or non-saturated alkyl; -$COR_6$; -O-alkyl; and -O-$COR_6$; -$R_7R_8$; $R_7N(R_6)_2$ and $R_7NHR_6R_8$; preferably containing 1, 2, 3, 4, 5, or 6 carbon atoms;
$R_5$ = branched or non-branched cyclic or non-cyclic, saturated or non-saturated alkyl; or

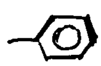 ,

preferably containing 1, 2, 3, 4, 5, or 6 carbon atoms;
$R_6$ = $C_1$ to $C_3$ alkyl;
$R_7$ = $C_1$ to $C_6$ alkyl, especially $C_1$, $C_2$ or $C_3$ alkyl;
$R_8$ = Epoxy, -$NH_2$ or -SH; especially

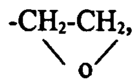 or 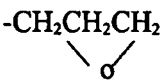

$R_9$ = Straight or branched $C_1$ to $C_6$ alkyl.

**9.** A method according to claim 8, wherein the sol is methyltrimethoxysilane (MeTMOS) or tetramethylsilicate (TMOS).

**10.** A method according to any preceding claim wherein the electrical potential applied to the electrically conductive surface is -900 to -1500 mV.

**11.** A method according to any preceding claim, wherein the electrical potential is applied for 20 to 120 seconds.

**12.** A method according to any preceding claim wherein the acidified sol suspension does not contain an alcohol and/or an electroreducer.

**13.** A method according to any preceding claim wherein an alcohol and/or an electroreducer is incorporated into the neutralised sol suspension.

**14.** A method according to any preceding claim comprising adding a silane coupling agent.

**15.** A method according to claim 14, comprising incorporating functionalised or non-functionalsied APTEOS the neutralised sol suspension.

**16.** A method according to claim 15, wherein the APTEOS is functionalised with a ferrocene, gluconamide or a lactiobionic group.

**17.** A method according to any preceding claim additionally comprising the addition of a mercaptan-containing silane and/or a bisfunctional silane.

**18.** A method according to any preceding claim, wherein the neutralised sol suspension additionally comprises one or more stabilisers.

**19.** A method according to claim 18 wherein the stabiliser is selected from a polyhydroxyalcohol, such as glycerol, polyethylene glycol or polyvinyl alcohol; polysaccharides, such as dextran or chitosan; polyalkylene imine; or sugars, such as mannitol, gluconate, lactitol or sucrose.

**20.** A method according to any one of claims 3 to 19, wherein the enzymes are selected from xanthine oxidase, glucose oxidase, lactate oxidase, cholesterol oxidase, galactose oxidase, glutamate oxidase, horse radish peroxidase, polyphenol oxidase, D-fructose dehydrogenase, L-glutamate dehydrogenase, alcohol dehydrogenase (such as methanol dehydrogenase), urease, uricase, lactate dehydrogenase, glutamic pyruvic transaminase, creatinase, sarcosine oxidase, glutaminase, nucleoside phosphorylase, ascorbate oxidase, cytochrome C oxidase, adenosine deaminase, D- or L-amino acid oxidase, tyrosinase and/or cholinedehydrogenase.

**21.** A method according to any one of claims 3 to 20, wherein two or more enzymes are used.

**22.** A method according to claim 21 wherein each enzyme is applied as a separate layer.

**23.** A method of producing a biological assay device, such as a biosensor or a microarray, comprising the use of a method as defined in any preceding claim to produce a layer of sol-gel containing a biological material onto a substrate.

**24.** A method according to claim 23, wherein the electrically conductive surface is an electrode.

**25.** A method according to claim 24, wherein the biosensor or microarray comprises two electrodes, each electrode having a layer of sol-gel having a different biorecognition element within it, formed by applying an electrical potential to a first electrode when in contact with a first neutralised sol suspension containing a first biorecognition element; and selectively applying an electrical potential to a second electrode, when the second electrode is in contact with a second neutralised sol suspension containing a second biorecognition element.

**26.** A microelectrode biological assay device obtainable by a method according to any one of claims 3 to 25, wherein the microelectrode is less than 10 mm long and is 10-50 μm in diameter, and comprises a layer of sol-gel on the surface of the microelectrode, the layer of sol-gel comprising biological material selected from an enzyme, antibody, fragment of an antibody, nucleic acid, polysaccharide, oligosaccharide, biomimetic polymer, virus or a whole cell.

**27.** A biological assay device according to claim 26, wherein the sol-gel is obtainable from a mixture comprising a mercaptan-containing silane and/or a bisfunctional silane.

**28.** In combination, a biological assay device according to claims 26 or 27 with a potentiometer.

**29.** Use of a biological assay device according to any of claims 26 or 27 or the combination according to claim 28, to detect one or more analytes.

## Patentansprüche

**1.** Verfahren zur Herstellung einer Schicht eines Sol-Gels auf einem Substrat, mit den Schritten:

(a) Bereitstellen einer gesäuerten Sol-Suspension;
(b) mindestens teilweise Neutralisieren der gesäuerten Suspension, um eine neutralisierte Sol-Suspension zu bilden;
(c) Kontaktieren einer elektrisch leitfähigen Oberfläche mit der neutralisierten Sol-Suspension; und
(d) Anlegen eines elektrischen Potenzials an die elektrisch leitfähige Oberfläche, um eine Schicht eines Sol-Gels auf der elektrisch leitfähigen Oberfläche zu bilden.

**2.** Verfahren nach Anspruch 1, zusätzlich mit dem Schritt des Zugebens eines oder mehrerer biologischer Materialien in die neutralisierte Sol-Suspension vor dem Anlegen des elektrischen Potenzials an die elektrisch leitfähige Oberfläche (Schritt C).

**3.** Verfahren nach Anspruch 2, bei welchem das biologische Material ausgewählt ist aus der Gruppe Enzym, Antikörper, Fragment eines Antikörpers, Nukleinsäure, Polysaccharide, Oligosaccharide, biomimetische Polymere, Virus, Mikroorganismus oder eine vollständige Zelle.

**4.** Verfahren nach einem der vorherigen Ansprüche, bei welchem die gesäuerte Sol-Suspension einen pH-Wert von weniger als pH 4 hat.

**5.** Verfahren nach einem der vorherigen Ansprüche, bei welchem die gesäuerte Sol-Suspension auf einen pH-Wert zwischen pH 5 und pH 7,5 neutralisiert wird.

**6.** Verfahren nach einem der vorherigen Ansprüche, bei welchem die gesäuerte Sol-Suspension durch Zugeben eines Puffers neutralisiert wird.

**7.** Verfahren nach einem der vorherigen Ansprüche, bei welchem das Sol ein Sol aus Alkoxysilan, Aluminiumoxid, Kolloidal-Metallhydroxid, Keramikoxid oder Zirkonia enthält.

**8.** Verfahren nach Anspruch 7, bei welchem das Sol die allgemeine Formel hat:

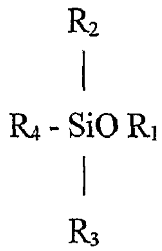

or $(R_9O)_3$-Si-$CH_2$-$CH_2$-Si-$(OR_9)_3$
worin:

$R_1$ = linear verkettetes, verzweigt verkettetes, zyklisches, nicht-zyklisches, gesättigtes oder nicht-gesättigtes, substituiertes oder nicht-substituiertes Alkyl; substituiertes oder nicht-substituiertes Aryl; -$NR_5$ und -$COR_6$, vorzugsweise enthaltend 1, 2, 3, 4, 5 oder 6 Kohlenstoffe;
$R_2$, $R_3$ und $R_4$ sind unabhängig ausgewählt aus linear verkettetem und verzweigt verkettetem, zyklischem oder nicht-zyklischem, gesättigtem oder nicht-gesättigtem Alkyl; -$COR_6$; -O-Alkyl; und -O-$COR_6$; -$R_7R_8$; $R_7N(R_6)_2$ und $R_7NHR_6R_8$; vorzugsweise enthaltend 1, 2, 3, 4, 5 oder 6 Kohlenstoff-Atome;
$R_5$ = verzweigtes oder nicht-verzweigtes, zyklisches oder nicht-zyklisches, gesättigtes oder nicht-gesättigtes Alkyl; oder

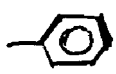,

vorzugsweise enthaltend 1, 2, 3, 4, 5 oder 6 Kohlenstoff-Atome;
$R_6$ = $C_1$ bis $C_3$ - Alkyl;
$R_7$ = $C_1$ bis $C_6$ - Alkyl, insbesondere $C_1$, $C_2$ oder $C_3$ - Alkyl;
$R_8$ = Epoxid, -$NH_2$ oder -SH; insbesondere

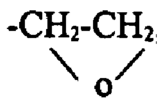 oder 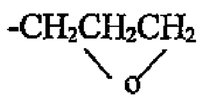 ;

$R_9$ = lineares oder verzweigtes $C_1$ bis $C_6$ - Alkyl.

**9.** Verfahren nach Anspruch 8, bei welchem das Sol Methyltrimethoxysilan (MeTMOS) oder Tetramethylsilikat (TMOS)

ist.

10. Verfahren nach einem der vorherigen Ansprüche, bei welchem das an die elektrisch leitfähige Oberfläche angelegte elektrische Potenzial -900 bis -1.500 mV ist.

11. Verfahren nach einem der vorherigen Ansprüche, bei welchem das elektrische Potenzial für 20 bis 120 Sekunden angelegt wird.

12. Verfahren nach einem der vorherigen Ansprüche, bei welchem die gesäuerte Sol-Suspension keinen Alkohol und/oder Elektroreducer enthält.

13. Verfahren nach einem der vorherigen Ansprüche, bei welchem ein Alkohol und/oder ein Elektroreducer in die neutralisierte Sol-Suspension eingebaut wird.

14. Verfahren nach einem der vorherigen Ansprüche, mit dem Zugeben eines Silanhaftvermittlers.

15. Verfahren nach Anspruch 14, mit dem Einbauen eines funktionalisierten oder nichtfunktionalisierten APTEOS in die neutralisierte Suspension.

16. Verfahren nach Anspruch 15, bei welchem die APTEOS mit einem Ferrocen, einem Gluconamid oder einer Lactiobionikgruppe funktionalisiert ist.

17. Verfahren nach einem der vorherigen Ansprüche, zusätzlich mit dem Zugeben eines Mercaptosilan und/oder eines bifunktionellen Silans.

18. Verfahren nach einem der vorherigen Ansprüche, bei welchem die neutralisierte Sol-Suspension zusätzlich einen oder mehrere Stabilisatoren enthält.

19. Verfahren nach Anspruch 18, bei welchem der Stabilisator ausgewählt ist aus der Gruppe Polyhydroxyalkohol, wie beispielsweise Glycerol, Polyethylenglykol oder Polyvinylalkohol; Polysaccharide, wie beispielsweise Dextran oder Chitosan; Polyalkylenimin; oder Zucker, wie beispielsweise wie Mannitol, Gluconat, Lactilol oder Succrose.

20. Verfahren nach einem der Ansprüche 3 bis 19, bei welchem die Enzyme ausgewählt sind aus der Gruppe Xanthinoxidase, Glukoseoxidase, Lactatoxidase, Cholesteroloxidase, Galactoseoxidase, Glutamatoxidase, Meerrettichperoxidase, Polyphenoloxidase, D-Fructose-Dehydrogenase, L- Glutamat-Dehydrogenase, Alkoholdehydrogenase (z.B. Methanoldehydrogenase), Urease, Uricase, Lactatdehydrogenase, Glutaminpyruvin-Transaminase, Creatinase, Sarcosinoxidase, Glutaminase, Nucleosid-Phosphorylase, Ascorbatoxidase, Cytochrome-C-Oxidase, Adenosin-Deaminase, D- oder L-Aminosäureoxidase, Tyrosinase und/oder Cholin-Dehydrogenase.

21. Verfahren nach einem der Ansprüche 3 bis 20, bei welchem zwei oder mehr Enzyme verwendet werden.

22. Verfahren nach Anspruch 21, bei welchem jedes Enzym als eine separate Schicht appliziert wird.

23. Verfahren zur Herstellung einer biologischen Analysevorrichtung, wie beispielsweise eines Biosensors oder einer Mikroanordnung, enthaltend die Verwendung eines Verfahrens nach einem der vorherigen Ansprüche, um eine Schicht des biologisches Material enthaltenden Sol-Gels auf einem Substrat zu erzeugen.

24. Verfahren nach Anspruch 23, bei welchem die elektrisch leitfähige Oberfläche eine Elektrode ist.

25. Verfahren nach Anspruch 24, bei welchem der Biosensor oder die Mikroanordnung zwei Elektrode enthält, wobei jede Elektrode eine Schicht des Sol-Gels mit einem unterschiedlichen Bioerkennungselement darin hat, gebildet durch Anlegen eines elektrischen Potenzials an eine erste Elektrode, während diese mit einer ersten neutralisierten Sol-Suspension enthaltend ein erstes Bioerkennungselement in Kontakt ist; und selektives Anlegen eines elektrischen Potenzials an eine zweite Elektrode, während die zweite Elektrode mit einer zweiten neutralisierten Sol-Suspension enthaltend ein zweites Bioerkennungselement in Kontakt ist.

26. Biologische Analysevorrichtung mit Mikroelektroden, erhältlich durch ein Verfahren nach einem der Ansprüche 3 bis 25, wobei die Mikroelektrode weniger als 10 mm lang ist und 10 bis 50 μm Durchmesser hat und eine Schicht

des Sol-Gels auf der Oberfläche der Mikroelektrode enthält, wobei die Schicht des Sol-Gels ein biologisches Material ausgewählt aus der Gruppe Enzym, Antikörper, Fragment eines Antikörpers, Nukleinsäure, Polysaccharide, Oligosaccharide, biomimetische Polymere, Virus, Mikroorganismus oder eine vollständige Zelle, enthält.

**27.** Biologische Analysevorrichtung nach Anspruch 26, bei welcher das Sol-Gel aus einer Mischung mit einem Mercaptosilan und/oder einem bifunktionalen Silan erhältlich ist.

**28.** Kombination einer biologischen Analysevorrichtung nach Anspruch 26 oder 27 mit einem Potentiometer.

**29.** Verwendung einer biologischen Analysevorrichtung nach einem der Ansprüche 26 oder 27 oder der Kombination nach Anspruch 28, um einen oder mehrere Analyten zu detektieren.

**Revendications**

**1.** Méthode de production d'une couche de sol-gel sur un substrat, comprenant les étapes consistant à :

(a) proposer une suspension sol acidifiée ;
(b) neutraliser au moins partiellement la suspension sol acidifiée pour former une suspension sol neutralisée ;
(c) mettre en contact une surface conductrice de l'électricité avec la suspension sol neutralisée ; et
(d) appliquer un potentiel électrique à la surface conductrice de l'électricité pour qu'une couche sol-gel se forme à la surface de la surface conductrice de l'électricité.

**2.** Méthode selon la revendication 1, comprenant en outre l'étape consistant à ajouter un ou plusieurs matériaux biologiques à la suspension sol neutralisée avant d'appliquer le potentiel électrique à la surface conductrice de l'électricité (étape C).

**3.** Méthode selon la revendication 2, dans laquelle le matériau biologique est sélectionné à partir d'une enzyme, d'un anticorps, d'un fragment d'anticorps, d'un acide nucléique, d'une polysaccharide, d'une oligosaccharide, de polymères biomimétiques, de virus, de microorganismes ou d'une cellule entière.

**4.** Méthode selon l'une des revendications précédentes dans laquelle la suspension sol acidifiée a un pH inférieur à pH 4.

**5.** Méthode selon l'une des revendications précédentes dans laquelle la suspension sol acidifiée est neutralisée à un pH entre 5 et 7,5.

**6.** Méthode selon l'une des revendications précédentes dans laquelle la suspension sol acidifiée est neutralisée par l'ajout d'une solution tampon.

**7.** Méthode selon l'une des revendications précédentes dans laquelle le sol comprend un sol d'alkoxysilane, d'alumine, d'hydroxyde de métal colloïdal, d'oxyde de céramique ou de zirconium.

**8.** Méthode selon la revendication 7, dans laquelle le sol a la formule générale suivante :

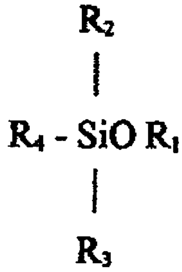

ou $(R_9O)_3$-Si-$CH_2$-$CH_2$-Si-$(OR_9)_3$
où :

$R_1$ chaîne droite, chaîne ramifiée, cyclique, non cyclique, saturée ou non saturée, à alkyle substitué ou non substitué ; à aryle substitué ou non substitué ; -NRs ; et $COR_6$ ; contenant de préférence 1, 2, 3, 4, 5 ou 6 carbones.

$R_2$, $R_3$ et $R_4$ sont sélectionnés indépendamment dans les composants suivants : chaîne droite et chaîne ramifiée, cyclique ou non cyclique, alkyle saturé ou non saturé ; $-COR_6$ ; -O-alkyle ; et $-O-COR_6$ ; $-R_7R_8$ ; $R_7N(R_6)_2$ et $R_7NHR_6R_8$ ; contenant de préférence 1, 2, 3, 4, 5 ou 6 atomes de carbone ;

$R_5$ alkyle ramifié ou non ramifié, cyclique ou non cyclique, saturé ou non saturé ; ou

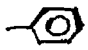 ,

contenant de préférence 1, 2, 3, 4, 5 ou 6 atomes de carbone ;

$R_6$ alkyle $C_1$ à $C_3$ ;

$R_7$ alkyle $C_1$ à $C_6$, en particulier alkyle $C_1$, $C_2$ ou $C_3$ ;

$R_8$ Epoxy, $-NH_2$ ou -SH ; en particulier

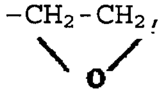 , ou 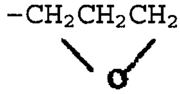

$R_9$ alkyle $C_1$ à $C_6$ droit ou ramifié.

**9.** Méthode selon la revendication 8, dans laquelle le sol est le méthyltriméthoxysilane (MeTMOS) ou le tétraméthylsilicate (TMOS).

**10.** Méthode selon l'une des revendications précédentes dans laquelle le potentiel électrique appliqué à la surface conductrice de l'électricité est compris entre -900 et -1500 mV.

**11.** Méthode selon l'une quelconque des revendications précédentes, dans laquelle le potentiel électrique est appliqué pendant 20 à 120 secondes.

**12.** Méthode selon l'une quelconque des revendications précédentes, dans laquelle la suspension sol acidifiée ne contient pas d'alcool ni d'électroréducteur.

**13.** Méthode selon l'une quelconque des revendications précédentes, dans laquelle un alcool et/ou un électroréducteur est incorporé dans la suspension sol neutralisée.

**14.** Méthode selon l'une quelconque des revendications précédentes, comprenant l'ajout d'un agent de couplage silane.

**15.** Méthode selon la revendication 14, comprenant l'incorporation d'APTEOS fonctionnalisée ou non fonctionnalisée à la suspension sol neutralisée.

**16.** Méthode selon la revendication 15, dans laquelle l'APTEOS est fonctionnalisée avec un groupe ferrocène, gluconamide ou lactiobionique.

**17.** Méthode selon l'une quelconque des revendications précédentes comprenant en outre l'ajout d'un silane contenant du mercaptan et/ou d'un silane bifonctionnel.

**18.** Méthode selon l'une quelconque des revendications précédentes, dans laquelle la suspension sol neutralisée comprend en outre un ou plusieurs stabilisateurs.

**19.** Méthode selon la revendication 18 dans laquelle le stabilisateur est sélectionné à partir d'un polyhydroxyalcool tel que le glycérol, le polyéthylène glycol ou l'alcool polyvinylique ; les polysaccharides, telles que le dextran ou la chitosane ; l'imine polyalkylène ou les sucres tels que le mannitol, le gluconate, le lactitol ou le sucrose.

**20.** Méthode selon l'une des revendications 3 à 19, dans laquelle les enzymes sont sélectionnées à partir de xanthine

oxydase, de glucose oxydase, de lactate oxydase, de cholestérol oxydase, de galactose oxydase, de glutamate oxydase, de peroxydase de raifort, de polyphénol oxydase, de déshydrogénase D-fructose, de déshydrogénase L-glutamate, de déshydrogénase alcool (par exemple la déshydrogénase de méthanol), d'uréase, d'uricase, de lactate déshydrogénase, de transaminase pyruvique glutamique, de créatinase, de sarcosine oxydase, de glutaminase, de phosphorylase nucléoside, d'ascorbate oxydase, de C cytochrome oxydase, d'adénosine désaminase, de D- ou L-amino acide oxydase, de tyrosinase et/ou de cholinédéshydrogénase.

**21.** Méthode selon l'une des revendications 3 à 20 dans laquelle deux ou plusieurs enzymes sont utilisées.

**22.** Méthode selon la revendication 21 dans laquelle chaque enzyme est appliquée sous forme de couche séparée.

**23.** Méthode de production d'un dispositif d'évaluation biologique tel que biocapteurs ou microréseau, comprenant l'utilisation d'une méthode telle que définie dans une revendication précédente pour produire une couche sol-gel contenant un matériau biologique sur un substrat.

**24.** Méthode selon la revendication 23, dans laquelle la surface conductrice de l'électricité est une électrode.

**25.** Méthode selon la revendication 24, dans laquelle le biocapteur ou le microréseau comprend deux électrodes, chaque électrode ayant une couche sol-gel contenant à l'intérieur un élément différent de bioreconnaissance, formée par l'application d'un potentiel électrique à une première électrode lorsqu'elle est en contact avec une première suspension sol neutralisée contenant un premier élément de bio reconnaissance ;
et
par l'application sélective d'un potentiel électrique à une seconde électrode lorsque la seconde électrode est en contact avec une seconde suspension sol neutralisée contenant un second élément de bio reconnaissance.

**26.** Dispositif d'évaluation biologique à microélectrode pouvant être obtenu par une méthode selon l'une des revendications 3 à 25, dans laquelle la microélectrode fait moins de 10 mm de long et 10-50 $\mu$m de diamètre et comprend une couche sol-gel sur la surface de la microélectrode, la couche sol-gel comprenant un matériau biologique sélectionné parmi une enzyme, un anticorps, un fragment d'anticorps, un acide nucléique, un polysaccharide, un oligosaccharide, un polymère biomimétique, un virus ou une cellule entière.

**27.** Dispositif d'évaluation biologique selon la revendication 26, dans lequel le sol-gel peut être obtenu à partir d'un mélange comprenant un silane contenant du mercaptan et/ou un silane bisfonctionnel.

**28.** En combinaison, dispositif d'évaluation biologique selon les revendications 26 ou 27 avec un potentiomètre.

**29.** Utilisation d'un dispositif d'évaluation biologique selon l'une des revendications 26 ou 27 ou combinaison selon la revendication 28, pour détecter un ou plusieurs analytes.

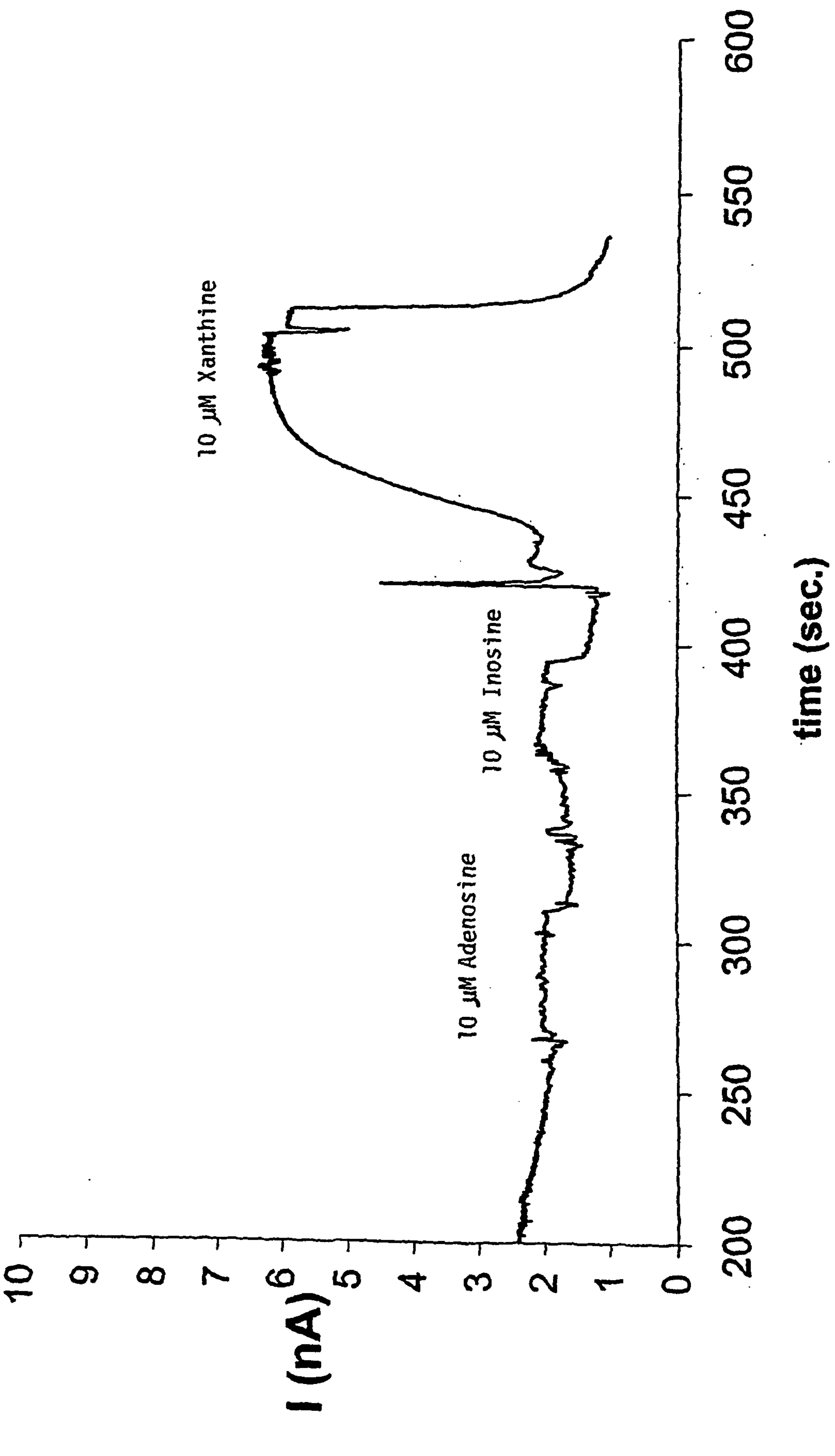
10 μM Xanthine
10 μM Inosine
10 μM Adenosine
I (nA)
0
1
2
3
4
5
6
7
8
9
10
time (sec.)
200
250
300
350
400
450
500
550
600

FIGURE 1

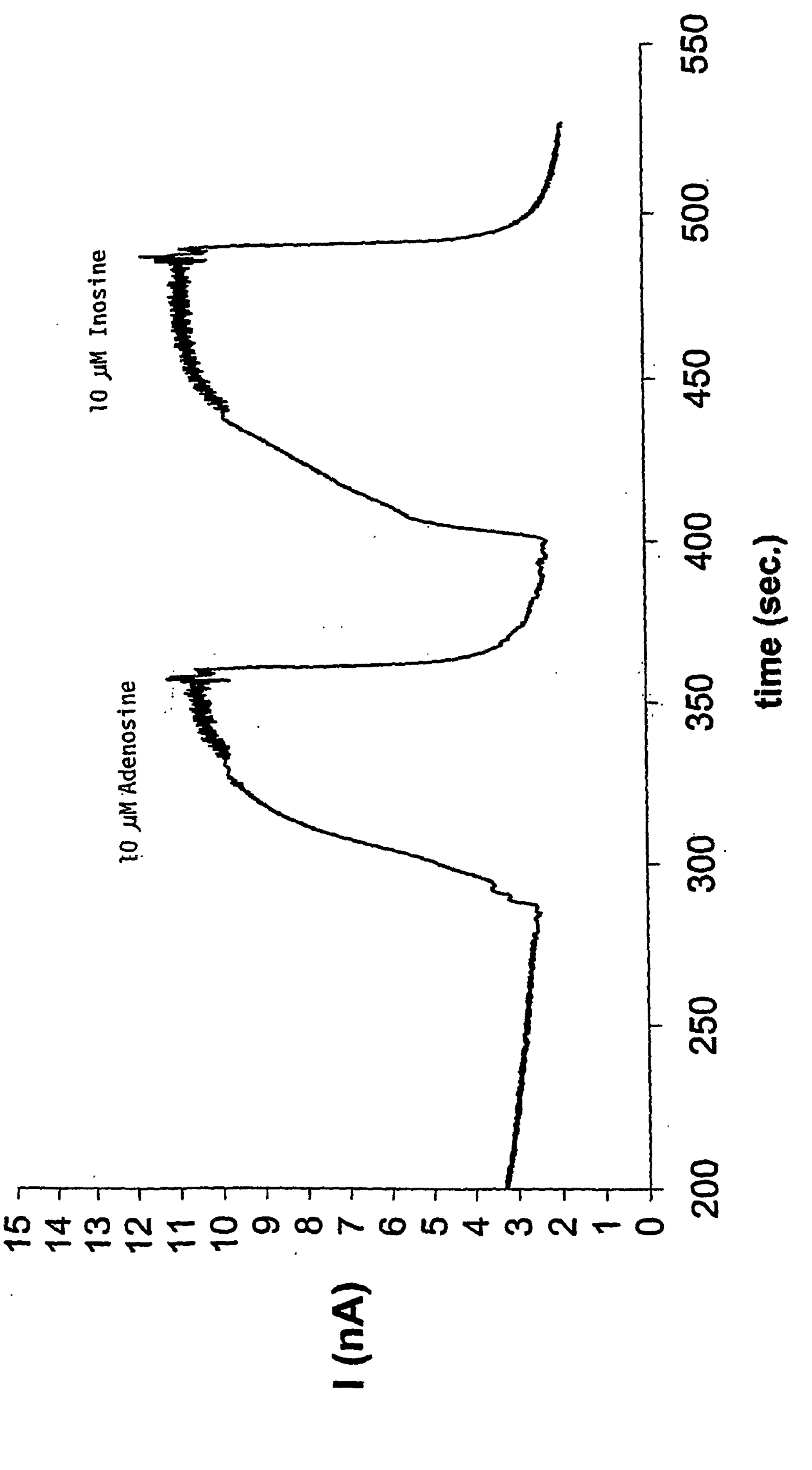

I (nA)
0 1 2 3 4 5 6 7 8 9 10 11 12 13 14 15
time (sec.)
200 250 300 350 400 450 500 550
10 µM Adenosine
10 µM Inosine


FIGURE 2

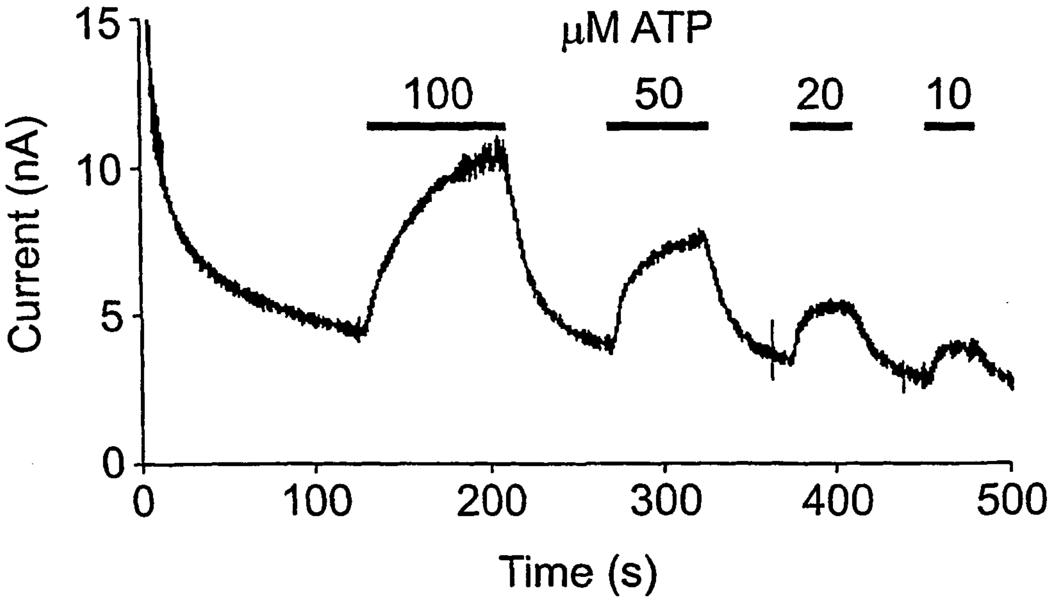
μM ATP
100
50
20
10
15
10
5
0
Current (nA)
0
100
200
300
400
500
Time (s)

FIGURE 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5698083 A
- EP 0537761 A
- US 6303290 B


### Non-patent literature cited in the description


- **Jones W.M. ; Fischbach (D.B.).** *J. Non-Crystalline Solids,* 1998, vol. 101, 123-126
- **Lillis B et al.** *Sensors and Activators,* 2000, vol. B68, 109-114
- **Black E. et al.** *J. Non. Cryst. Solids,* 2001, vol. 285, 1-3
- **Palmisano F. et al.** *Fresenius J. Anal. Chem.,* 2000, vol. 366, 586-601
- **Burmister J.J. ; Gerhardt G.A.** *Anal. Chem.,* 2001, vol. 73, 1037-1042
- **Screenivas G. et al.** *Anal. Chem.,* 1996, vol. 68, 1858-1864
- **Shacum R. et al.** *Adv. Mater,* 1999, vol. 11 (5), 384-388
- **Templin M. Weld.** *Trends in Biotechnology,* 2002, vol. 20, 160-165
- **Angenendt P et al.** *Anal. Biochem.,* 2002, vol. 309, 253-260
- **Gheorghies C et al.** *Analele Stiintifice Ale Universitatii, Tomul XLV - XLVI, s. Fizica Starii Condensate,* 1999, 268-275
- *Discover,* 1998, vol. 19